# EUROPÄISCHE PATENTSCHRIFT

(11) **EP 3 473 150 B1**
(45) Veröffentlichungstag und Bekanntmachung des Hinweises auf die Patenterteilung: **26.01.2022**
(21) Anmeldenummer: 18000777.5
(22) Anmeldetag: 28.09.2018
(51) Int. Cl.: A47K 10/48, A61L 2/10

(54) **HANDTROCKNER MIT UV-DESINFEKTIONSVORRICHTUNG**
HAND DRYER WITH UV DISINFECTION DEVICE
SÈCHE-MAINS AVEC UN DISPOSITIF DE DÉSINFECTION PAR UV

(30) Priorität: 17.10.2017 DE 102017009637
(43) Veröffentlichungstag der Anmeldung: 24.04.2019
(73) Patentinhaber: Ushio Germany GmbH, 85643 Steinhöring (DE)
(72) Erfinder: Hishinuma, Nobuyuki, 85643 Steinhöring (DE); Wakahata, Yasuhiko, 85643 Steinhöring (DE)
(74) Vertreter: Tomerius, Isabel

(56) Entgegenhaltungen:
- JP-A- 2005 305 031
- JP-A- 2006 238 940
- Anonymous: "Ultraviolet germicidal irradiation - Wikipedia", , 15. Februar 2019 (2019-02-15), XP055559462, Gefunden im Internet: URL:https://en.wikipedia.org/wiki/Ultravio let_germicidal_irradiation [gefunden am 2019-02-20]

## Beschreibung

Die Erfindung betrifft einen Handtrockner, in dem durch eine Gehäuseöffnung eingeschobene Hände in einem Hohlraum mittels einer Luftströmung getrocknet werden können. Derartige Handtrockner sind im Stand der Technik grundsätzlich bekannt. Sie umfassen üblicherweise einen Lüfter oder ein Gebläse, mit dem Luft durch eine oder mehrere Düsen auf die in den Hohlraum eingeschobenen Hände geblasen wird und diese so getrocknet werden. In der WO 2010/015040 A1 beispielsweise ist ein Handtrockner beschrieben, bei dem eine Luftströmung in Form eines dünnen Luftvorhangs mit hoher Geschwindigkeit aus schlitzförmigen Düsen auf die Hände geblasen wird. Die von den Händen abgeblasene Flüssigkeit wird in einem Sammelbereich aufgefangen. Die zum Trocknen verwendete Luft dagegen wird teilweise innerhalb des Handtrockners rezirkuliert und gelangt teilweise in die Umgebung des Handtrockners. Auf diese Weise können jedoch an den Händen anhaftende Verunreinigungen und insbesondere Keime wie Sporen oder Bakterien von der Luft mitgeschleppt werden und auf diese Weise in die Umgebungsluft gelangen.

In vielen Bereichen ist die Anreicherung der Luft mit Verunreinigungen und insbesondere Keimen nicht erwünscht oder sogar unzulässig. Verwiesen werden kann in diesem Zusammenhang beispielsweise auf Krankenhäuser oder Pflegeheime, Produktionsstätten, in denen erhöhte Anforderungen an saubere Umgebungsbedingungen gestellt werden, beispielsweise die Arzneimittel- oder Lebensmittelproduktion, oder die Gastronomie. Hierzu gibt die JP 2006 238 940 A beispielsweise an, dass hygienisch relevante Flächen, wie an Waschbecken und Handtrocknern, mit photokatalytischen Substanzen beschichtet und dann, wenn kein Benutzer in der Nähe ist, mit UV-Licht bestrahlt werden können. Auf diese Weise sind die Oberflächen insgesamt sauberer. Unabhängig von diesen speziellen Bereichen ist allgemein eine erhöhte Anforderung an gute Hygienebedingungen festzustellen.

Zusätzlich zur Reinhaltung der Umgebung ist in vielen Bereichen auch eine Desinfektion oder sogar Sterilisation der Hände und gegebenenfalls auch der Arme derjenigen Personen erforderlich, die in solchen Bereichen arbeiten. Nach dem normalen Händewaschen verbleiben immer noch etwa 5 % der vor dem Händewaschen auf den Händen befindlichen Keime auf der Haut. Um diese Keimzahl zu reduzieren, sind also zusätzliche Maßnahmen erforderlich. Diese bestehen üblicherweise im Waschen mit keimtötenden Substanzen, beispielsweise flüssigen oder gelartigen Zusammensetzungen auf Alkoholbasis. Damit eine vollständige Desinfektion oder Sterilisation erreicht wird, ist aber ein sehr gründliches Vorgehen erforderlich, das eine lange Zeit in Anspruch nimmt. Die nötige Gründlichkeit fehlt in vielen Fällen, was zu einer Infektionsgefahr durch auf Händen und Armen verbliebene Keime führt.

Zur Verringerung der Keime beim Trocknen der Hände wurde in der DE 102012008253 A1, JP 2005 305 031 A und EP 2656762 A2 bereits ein Handtrockner vorgeschlagen, der verschiedene Mittel zur Keimreduktion, unter anderem in Form einer UV-Strahlung emittierenden Lampe, umfasst. Genauere Angaben dazu werden in der Anmeldung nicht gemacht. Allerdings haben die dieser Erfindung vorausgegangenen Untersuchungen gezeigt, dass nur die Verwendung bestimmter UV-Lampen und eine bestimmte Anordnung derselben zu der gewünschten Keimreduktion ohne Gefährdung der Gesundheit des Benutzers führen.

Aufgabe der Erfindung ist es entsprechend, einen Handtrockner anzugeben, der die Anzahl von Keimen auf den Händen des Benutzers und in der bei dem Trockenvorgang rezirkulierten und/oder in die Umgebung gelangenden Luftströmung reduziert, ohne dabei zu einer gesundheitlichen Belastung des Benutzers zu führen.

Die Lösung dieser Aufgabe gelingt mit dem Handtrockner gemäß Anspruch 1. Bevorzugte Weiterbildungen sind den Unteransprüchen zu entnehmen.

Die Erfindung betrifft in ihrem breitesten Aspekt also einen Handtrockner mit einem Gehäuse, in dem ein durch eine Gehäuseöffnung von außen zugänglicher Hohlraum zur Aufnahme mittels einer Luftströmung im Hohlraum zu trocknender Hände ausgebildet ist, und mit einer Vorrichtung zur Erzeugung der Luftströmung sowie einer Vorrichtung zur Erzeugung von UV-Strahlung, welche wenigstens eine im ultravioletten Wellenlängenbereich emittierende Lampe umfasst, die derart im Gehäuse angeordnet ist, dass sie UV-Strahlung in den Hohlraum abgibt. Die Vorrichtung zur Erzeugung von UV-Strahlung ist derart ausgebildet, dass die in den Hohlraum abgegebene UV-Strahlung mit einer Wellenlänge im Bereich von 228 bis 380 nm eine Intensität von maximal 20 % der Intensität der in den Hohlraum abgegebenen UV-Strahlung mit einer Wellenlänge im Bereich von 200 bis 380 nm besitzt.

Der erfindungsgemäße Handtrockner verwendet eine Vorrichtung zur Erzeugung von UV-Strahlung, welche ein spezielles Spektrum der Strahlung in den Hohlraum abgibt. Das Strahlungsspektrum ist so optimiert, dass die den Hohlraum erreichende UV-Strahlung mit einem Wellenlängenbereich von 200 bis 380 nm ganz überwiegend, nämlich zu mindestens 80 %, im Wellenlängenbereich unter 228 nm liegt. Lediglich maximal 20 % der Intensität der in den Hohlraum abgegebenen UV-Strahlung (womit hier immer die Strahlung im Bereich von 200 bis 380 nm bezeichnet wird) fällt in den Wellenlängenbereich von 228 bis 380 nm. Bevorzugt macht die Intensität der UV-Strahlung in letztgenannten Bereich maximal 15 %, besonders bevorzugt maximal 10 % und insbesondere maximal 7 % der Intensität der in den Hohlraum abgegebenen UV-Strahlung (200 bis 380 nm) aus.

Das im erfindungsgemäßen Handtrockner eingesetzte UV-Spektrum mit einem erhöhten Strahlungsanteil im Wellenlängenbereich unter 228 nm und einer reduzierten Intensität im Bereich von 228 bis 380 nm führt zu einer beinahe vollständigen Abtötung der auf der Haut befindlichen Keime, insbesondere zur praktisch vollständigen Beseitigung von Bakterien, darunter auch multiresistenten Bakterien. Andererseits wird die in den Hohlraum abgegebene UV-Strahlung weder von menschlicher DNA noch RNA absorbiert und wirkt damit nicht erbgutschädigend auf menschliche Zellen. Auch Augenschäden treten in diesem Wellenlängenbereich nicht auf. Würde dagegen das gesamte Spektrum der UV-Strahlung in den Hohlraum abgestrahlt, wäre dies nicht der Fall. Besonders im Wellenlängenbereich oberhalb von 250 nm wird UV-Strahlung sowohl von menschlicher RNA als auch DNA absorbiert und kann zu Strangbrüchen und zur anschließenden Bildung von Doppelbindungen oder Dimeren führen. Auch zahlreiche menschliche Proteine absorbieren UV-Strahlung im höheren UV-C-Wellenlängenbereich und können, wie auch das menschliche Auge, durch diese geschädigt werden. Die erfindungsgemäß eingesetzte Vorrichtung zur Erzeugung von UV-Strahlung gibt daher gezielt UV-Strahlung im niedrigen UV-C-Bereich ab, während zum kritischen Wellenlängenbereich oberhalb von 250 nm gezielt ein hinreichender Abstand gehalten und die Intensität der UV-Strahlung im Wellenlängenbereich von 228 bis 380 nm maximal 20 % der gesamten UV-Strahlung im Wellenlängenbereich von 200 bis 380 nm beträgt. Die Gesundheitsrisiken für die Benutzer des erfindungsgemäßen Handtrockners können auf diese Weise auf ein Minimum reduziert werden, während Keime und insbesondere Bakterien praktisch vollständig abgetötet werden. Dies gilt nicht nur für auf den Händen des Benutzers befindliche Keime, sondern auch für solche, die mit der Luftströmung im Handtrockner mitgeführt werden.

Das vorstehend beschriebene Strahlungsspektrum kann erfindungsgemäß auf unterschiedliche Art und Weise erreicht werden. In einer ersten Möglichkeit wird als Lampe eine solche ausgewählt, welche eine Haupt-Emissionswellenlänge von unter 228 nm aufweist. Bevorzugt wird dabei eine solche Lampe eingesetzt, welche bereits von sich aus und ohne weitere Maßnahmen die erfindungsgemäße Intensitätsverteilung der UV-Strahlung bereitstellt. Besonders geeignet sind hier für dielektrische Barrierentladungslampen und zwar insbesondere KrCl- und KrBr-Excimer-Barrierentladungslampen. Die Haupt-Wellenlängen der beiden Excimer-Barrierentladungslampen liegen bei 222 nm bzw. 207 nm. Oberhalb von 228 nm geben beide Lampentypen nur noch minimal ultraviolette Strahlung ab. Für den Einsatz im erfindungsgemäßen Handtrockner stellen diese beiden Excimer-Barrierentladungslampen daher besonders geeignete Beispiele dar.

Eine zweite Möglichkeit zur Erzeugung des gewünschten UV-Spektrums besteht in der Verwendung einer Lampe, welche Strahlung erzeugt, die ein Fluoreszenzmaterial anregt, welches wiederum Strahlung mit einer Hauptwellenlänge unter 228 nm aufweist. Derartige Lampen, bei denen die ursprünglich erzeugte Strahlung mittels eines Fluoreszenzmaterials in Strahlung einer anderen Wellenlänge konvertiert wird, sind grundsätzlich bekannt. Erfindungsgemäß werden zur Erzeugung der anregenden Strahlung bevorzugt Edelgas-Barrierentladungslampe verwendet. Besonders geeignet sind hierfür Xenon-, Krypton- und Argon-Barrierentladungslampen. Als Fluoreszenzmaterial (UV-C-emittierender Leuchtstoff) kann beispielsweise Lanthanphosphat-Praseodym, LaPO₄:Pr, eingesetzt werden.

Eine weitere Möglichkeit zur Erzeugung des gewünschten UV-Spektrums besteht im Einsatz wenigstens eine Lampe, welche UV-Strahlung mit einem breiteren als dem gewünschten Wellenlängenbereich erzeugt, wobei die nicht erwünschten Wellenlängenbereiche mittels eines Filtermaterials herausgefiltert werden. Derartige Filtermaterialien sind ebenfalls grundsätzlich bekannt. Bevorzugt werden Filter eingesetzt, welche UV-Strahlung mit einer Wellenlänge im Bereich von 228 bis 300 nm und bevorzugt 230 bis 260 nm herausfiltern. Das Herausfiltern von UV-Strahlung mit etwa 254 nm ist besonders wichtig, da letztere Wellenlänge zahlreiche menschliche Proteine sowie menschliche RNA und DNA schädigen kann.

Die vorstehend beschriebenen Möglichkeiten zur Erzeugung des gewünschten UV-Spektrums können für sich allein oder in beliebigen Kombinationen untereinander eingesetzt werden. So ist es insbesondere möglich, die in der ersten und zweiten beschriebenen Variante gezielt aufgrund ihres Emissionsspektrums gewählten Lampentypen in Kombination mit einem geeigneten Filter zu verwenden. Auf diese Weise lässt sich ein geringer Anteil an UV-Strahlung im Wellenlängenbereich von 228 bis 380 nm realisieren.

Das Filtermaterial kann auf jede geeignete Art und Weise in der Vorrichtung zur Erzeugung von UV-Strahlen angeordnet werden. Eine Möglichkeit besteht darin, das Filtermaterial auf den Lampenkolben als Beschichtung aufzutragen oder in das Material des Lampenkolbens einzuarbeiten. Geeignete Filtermaterialien können Hafniumoxid und/oder Siliciumoxid enthalten. Bevorzugt ist ein Filtermaterial, das abwechselnde Schichten aus Hafniumoxid und Siliciumoxid enthält.

Die wenigstens eine Lampe der Vorrichtung zur Erzeugung von UV-Strahlung kann auf verschieden Art und Weise im Handtrockner angeordnet werden. In einer bevorzugten Variante ist der Lampenkolben der Lampe so im Gehäuse angeordnet, dass ein Teil desselben in den Hohlraum hineinragt, wobei ein überwiegender Teil des Lampenkolbens sich außerhalb des Hohlraums befindet. Anders als in einem Fall eines bündig mit der den Hohlraum begrenzenden Wand abschließenden Lichtaustrittsfensters, hinter dem die Lampe angeordnet ist, kann die UV-Strahlung den gesamten Hohlraum besser erreichen und diesen insgesamt von Keimen befreien. Ein Großteil des Lampenkolbens ist jedoch weiterhin außerhalb des Hohlraums angeordnet und so gut vor Beschädigung und gegen Verunreinigung durch Schmutzwasser geschützt. Unter einem "Großteil des Lampenkolbens" ist im Rahmen dieser Erfindung ein vom Lampenkolben eingenommenes Volumen zu verstehen, das mehr als 50 % des Gesamtvolumens des Lampenkolbens ausmacht. Bevorzugt ist es, wenn nicht mehr als 30 % des Lampenkolben-Volumens in den Hohlraum vorstehen.

In einer anderen Variante befindet sich die wenigstens eine Lampe mit ihrem gesamten Lampenkolben vollständig im Hohlraum. Diese Möglichkeit kommt vor allem dann in Betracht, wenn der Hohlraum ausreichend groß ist, um die wenigstens eine Lampe aufzunehmen und dabei genug Platz für die Hände des Benutzers zu lassen. Von Vorteil werden in dieser Variante Lampen mit einem röhrenförmigen Lampenkolben geringen Durchmessers verwendet.

Grundsätzlich kann bereits eine UV-Lampe im erfindungsgemäßen Handtrockner ausreichen. Bevorzugt ist es jedoch, wenn wenigstens zwei Lampen auf gegenüberliegenden Seiten des Hohlraums angeordnet sind und die Hände des Benutzers von zwei Seiten mit UV-Strahlung bestrahlen. Besitzt die wenigstens eine Lampe einen röhrenförmigen Lampenkolben, wird dieser bevorzugt im Wesentlichen parallel zur Öffnungsebene der Gehäuseöffnung angeordnet. "Im Wesentlichen parallel" bedeutet dabei eine Neigung von maximal 15° bezüglich der Öffnungsebene. Bevorzugt beträgt die Neigung nicht mehr als 5° und insbesondere nicht mehr als 2° zur Öffnungsebene. Alternativ können auch Lampen mit im Wesentlichen kugel- oder eiförmigem Lampenkolben eingesetzt werden. Dabei können auch mehrere Lampen pro Hohlraum-Seite nebeneinander angeordnet werden. Bezüglich ihrer Neigung zur Öffnungsebene der Gehäuseöffnung gilt das für röhrenförmige Lampenkolben Gesagte entsprechend.

In einer bevorzugten Ausführungsform der Erfindung ist der im Hohlraum befindliche Teil des Lampenkolbens von einem Fensterbauteils gegen den Hohlraum des Handtrockners abgegrenzt. Das Fensterbauteil dient als Schutz für die Lampe und schließt den Hohlraum vorzugsweise luft- und feuchtigkeitsdicht gegenüber dem Raum ab, in dem die Lampe angeordnet ist. Wird mehr als eine Lampe verwendet, kann jede für sich von einem Fensterbauteil geschützt sein, oder es kann ein Fensterbauteil für mehrere Lampen verwendet werden. Wird ein Filtermaterial zum Herausfiltern nicht gewünschter UV-Wellenlängenbereiche verwendet, kann dieses beispielsweise als Beschichtung auf dem Fensterbauteil vorgesehen sein. Alternativ ist es möglich, das Fensterbauteil ganz oder teilweise aus einem Filtermaterial herzustellen. Selbstverständlich können auch Filtermaterialien sowohl auf/in dem Lampenkolben als auch dem Fensterbauteil eingesetzt werden. Zudem können mit dem Filtermaterial auch andere Wellenlängenbereiche außerhalb des Ultraviolettbereichs ausgefiltert werden. Hinsichtlich geeigneter Materialien kann auf das vorstehend Beschriebene verwiesen werden.

Damit ein möglichst großer Teil der von der Vorrichtung zur Erzeugung von UV-Strahlung erzeugten Strahlung in den Hohlraum des Handtrockner gelangt, ist es bevorzugt, den Lampenkolben unter Freilassung einer Strahlungsaustrittsöffnung mit einem Reflektor zu umgeben, welcher die UV-Strahlung mit einer Wellenlänge von unter 228 nm in Richtung auf den Hohlraum reflektiert. Je nach Art der verwendeten Lampe kann dabei natürlich auch Strahlung mit anderen Wellenlängen vom Reflektor reflektiert werden, wobei dann nicht gewünschte UV-Strahlung gegebenenfalls mittels eines Filtermaterials abgetrennt wird. Als Reflektor kann grundsätzlich jeder aus dem Stand der Technik bekannte und geeignete Reflektor eingesetzt werden. Beispielsweise kann es sich um eine Metallbeschichtung des Lampenkolbens handeln. Alternativ kommt auch eine Metallhülse in Betracht, die unter Freilassung der Strahlungsaustrittsöffnung auf der Außenseite des Lampenkolbens angeordnet ist. Im Falle dielektrischer Barrierentladungslampen kann die Metallhülse gleichzeitig auch als Außenelektrode der Lampe verwendet werden. Der Reflektor sorgt nicht nur für eine effektive Abgabe der UV-Strahlung in den Hohlraum, sondern verhindert auch, dass UV-Strahlung in das Innere des Gehäuses gelangt, wenn die Lampe teilweise außerhalb des Hohlraums liegt, und die dort angeordneten Komponenten schädigt. Auf diese Weise kann die Lebensdauer des Handtrockners verlängert werden.

Als zweckmäßig hat es sich erwiesen, die Strahlungsaustrittsöffnung so auszubilden, dass Strahlung mit einem Öffnungswinkel von mindestens 30° in Richtung des Hohlraums abgegeben wird. Dies gewährleistet eine ausreichend große Bestrahlungsfläche und somit eine im Wesentlichen vollständige Bestrahlung der in den Hohlraum eingeschobenen Hände eines Benutzers.

Der Grundaufbau des erfindungsgemäßen Handtrockners kann grundsätzlich dem Aufbau herkömmlicher Handtrockner entsprechen und sich von diesen nur durch die Vorrichtung zur Erzeugung von UV-Strahlung unterscheiden. Erfindungsgemäß bevorzugt werden solche Handtrockner verwendet, die in ihrem Grundaufbau denjenigen entsprechen, die in der DE 102012008253 A1, EP 2656762 A2 und WO 2007/015040 A1 beschrieben sind. Dies betrifft insbesondere die Zufuhr von Luft in den Trocknungshohlraum. Erfindungsgemäß werden hierfür also ebenfalls bevorzugt Flachdüsen verwendet, mit denen dünne Luftvorhänge mit hoher Geschwindigkeit, bevorzugt einer Geschwindigkeit von mindestens 15 m/s, auf die zu trocknenden Hände geblasen werden. Zweckmäßig weist ein erfindungsgemäßer Handtrockner also zwei sich an den Längsöffnungsseiten der Gehäuseöffnung gegenüberliegende Flachdüsen auf, mit denen die Luftströmung in den Hohlraum eingeblasen wird. Als besonders bevorzugt hat sich dabei erwiesen, wenn die Düsenöffnungen einen Neigungswinkel gegenüber der Öffnungsfläche der Gehäuseöffnung von wenigstens 7 ° aufweisen. Auf diese Weise wird die in den Hohlraum eingeblasene Luft schräg und mit relativ geringem Winkel auf die zu trocknenden Hände und an diesen entlang geführt. Als Vorrichtung zur Erzeugung der Luftströmung kann in an sich bekannter Weise ein Gebläse oder ein Lüfter verwendet werden.

Bezüglich der Flachdüse, mit welcher Luft in den Hohlraum eingeblasen wird, ist die wenigstens eine Lampe zweckmäßig so angeordnet, dass sie sich innerhalb eines streifenförmigen Bereichs befindet, der auf einer den Hohlraum begrenzenden Seitenwand gegenüber der Flachdüse liegt. Oberer und unterer Rand des streifenförmigen Bereichs entsprechen Schnittlinien, mit denen Grenzlinien eines Winkelbereichs die Seitenwand schneiden, wobei der Winkelbereich von der Luftaustrittsrichtung aus der Flachdüse als Mittelebene ausgeht und sich plus und minus 45° beidseitig der Mittelebene erstreckt. Die Lampe wird in Bezug auf die Flachdüse also so auf der gegenüberliegenden Seite des Hohlraums ausgerichtet, dass die von den Händen des Benutzers abgeblasene Feuchtigkeit und die in ihr enthaltenen Keime auf die Lampe hin geblasen und so mit großer Sicherheit von der UV-Strahlung getroffen wird.

Die Führung der Luftströmung im Handtrockner kann ebenfalls grundsätzlich auf bekannte Art und Weise erfolgen. In einer Variante wird die dem Hohlraum zugeführte Luft nicht rezirkuliert und entweicht aus dem Hohlraum in die Umgebung des Handtrockners. In einer anderen Variante wird wenigstens ein Teil der in den Hohlraum zum Trocknen der Hände eingeblasenen Luft rezirkuliert und dem Hohlraum erneut zugeführt. Zu diesem Zweck weist der erfindungsgemäße Handtrockner dann wenigstens einen Abluftkanal und wenigstens einen Zuluftkanal auf, die mit dem Hohlraum derart in Verbindung stehen, dass die Luftströmung durch Abluft- und Zuluftkanal sowie den Hohlraum zirkuliert werden kann. Die Vorrichtung zur Erzeugung der Luftströmung - wie Gebläse oder Lüfter - kann prinzipiell an beliebiger Stelle innerhalb dieses Lüftungskreislaufes angeordnet werden, also in Abluftkanal, Zuluftkanal oder im Bereich des Hohlraums. Bevorzugt ist es, die Vorrichtung zur Erzeugung der Luftströmung im Bereich des Abluftkanals, durch den die Luftströmung aus dem Hohlraum herausgeführt wird, anzuordnen.

Um das Herausgelangen von Keimen aus dem Bereich des Handtrockners in dessen Umgebung möglichst zu vermeiden, kann eine möglichst geschlossene Bauweise des erfindungsgemäßen Handtrockners von Vorteil sein (vergleiche DE 102012008253 A1 und EP 2656762 A2). Andere bekannte Handtrockner, beispielsweise der in der WO 2007/015040 A1 beschriebene, weisen dagegen eine vergleichsweise offene Bauweise auf, die den Zweck hat, einen Staudruck zu vermeiden und Luft schnell wegströmen zu lassen. Aus diesem Grund ist beispielsweise der zum Trocknen der Hände bestimmte Hohlraum seitlich offen ausgebildet. Im Rahmen der Erfindung können grundsätzlich beide beschriebenen Bautypen zum Einsatz kommen.

Unabhängig von der Bauweise des Handtrockners kann eine weitere Verbesserung im Hinblick auf eine Keimreduktion erfindungsgemäß dadurch erreicht werden, dass zumindest ein Teil der den Hohlraum begrenzenden Wände wenigstens bereichsweise mit einem Fotokatalysator versehen ist. Dabei handelt es sich um eine Verbindung, die unter dem Einfluss von Strahlung in der Lage ist, organische Verbindungen zu zersetzen. Konkret wird im vorliegenden Fall ein Fotokatalysator eingesetzt, der bei Bestrahlung mit der von der Vorrichtung zur Erzeugung von UV-Strahlung erzeugten Strahlung zur Abtötung von Keimen im Hohlraum beiträgt. Dies geschieht beispielsweise dadurch, dass der Fotokatalysator unter dem Einfluss von UV-Strahlung Ozon und/oder Radikale - wie beispielsweise aus Wasser gebildete OH-Radikale - freisetzt, welche mit den Keimen reagieren und diese zerstören. Ein bevorzugter Fotokatalysator ist Titandioxid, das beispielsweise in einer auf die Oberfläche der den Hohlraum umgebenden Wände aufgetragenen Beschichtung enthalten oder in das Material der Wände eingearbeitet ist. Um eine möglichst vollständige Abtötung von Keimen auf den Hohlraum-Wänden zu erreichen, sind diese möglichst vollständig mit dem Fotokatalysator versehen oder beschichtet.

Die Erfindung soll nachfolgend anhand von Zeichnungen näher erläutert werden. In den rein schematischen Zeichnungen sind gleiche Teile mit gleichen Bezugszeichen benannt. In den Figuren zeigen:
- Fig. 1: ein Ausführungsbeispiel eines erfindungsgemäßen Handtrockners;
- Fig. 2: den Handtrockner gemäß Figur 1 in einem Querschnitt entlang der Linie X-X;
- Fig. 2a: eine Ausschnittvergrößerung des Bereichs Y aus Figur 2;
- Fig. 2b: die Anordnung einer Lampe in Bezug auf eine Flachdüse im Handtrockner gemäß Figuren 1 und 2;
- Fig. 3: den Handtrockner gemäß Figur 1 in Seitenansicht während der Verwendung durch einen Benutzer;
- Fig. 4a: die Anordnung einer Lampe bezüglich des Hohlraums und einer den Hohlraum begrenzenden Seitenwand im Querschnitt senkrecht zur Längsachse der Lampe;
- Fig. 4b: eine alternative Lampenordnung in eine Figur 4a entsprechenden Ansicht;
- Fig. 5: eine alternative Ausführungsform des Handtrockners gemäß Figur 1 in einem Querschnitt entlang der Linie X-X;
- Fig. 5a-c: verschiedene Lampenanordnungen im Bereich des Ausschnitts Z aus Figur 5;

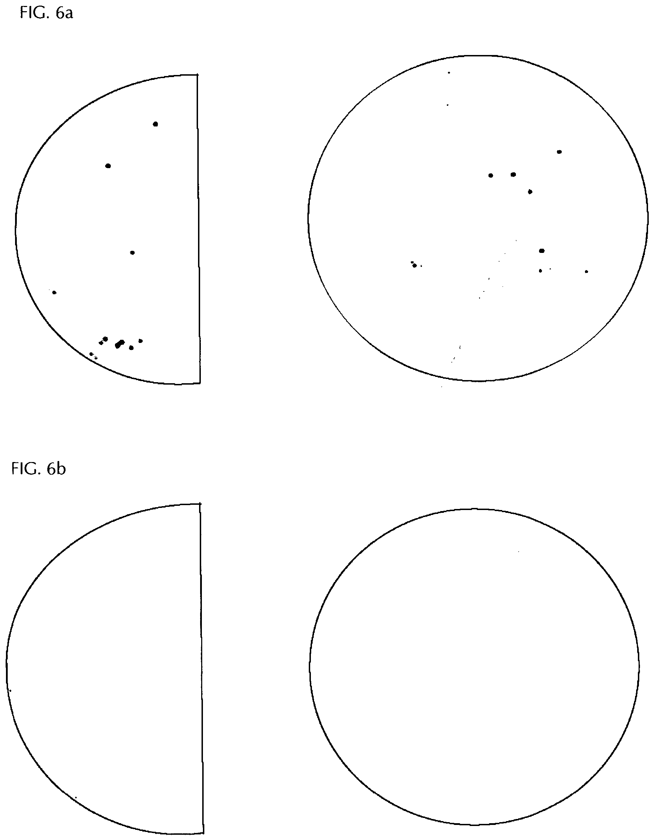

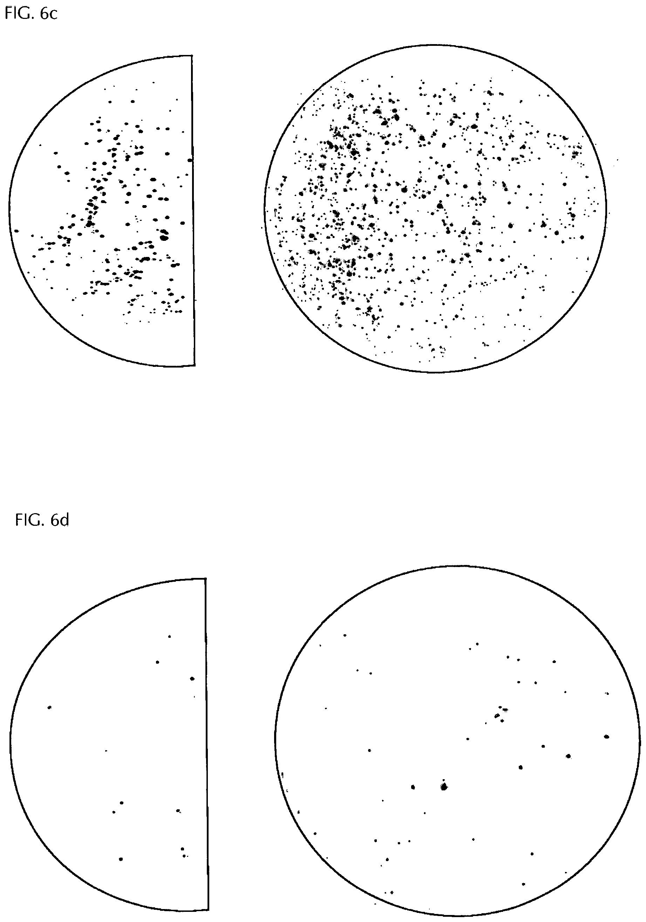

- Fig. 6a-e: Draufsichten auf Petrischalen, in denen Proben kultiviert worden, die nach verschiedenen nicht beanspruchten Wasch- und Sterilisationsmethoden von der Hand einer Testperson abgenommen wurden.

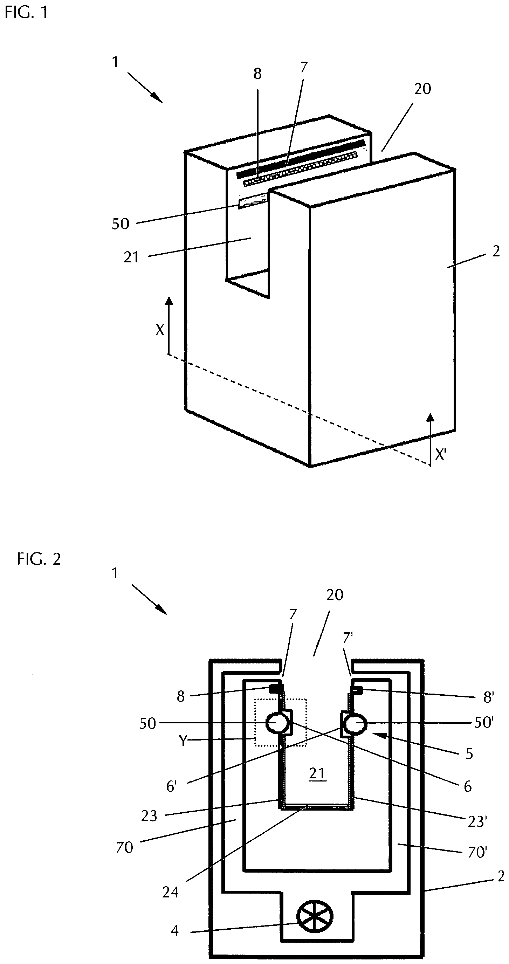

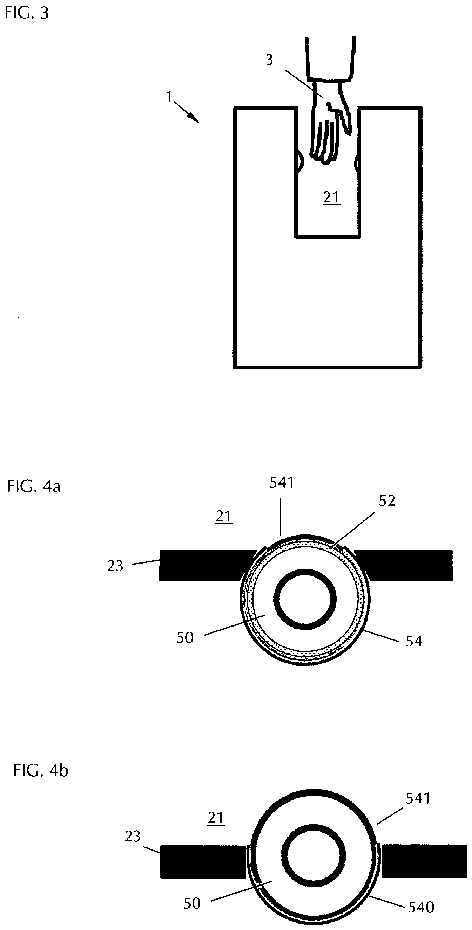

Figur 1 zeigt die stark vereinfachte perspektivische Darstellung eines erfindungsgemäßen Handtrockners 1 mit einem Gehäuse 2, das auf seiner Oberseite eine die gesamte Gehäusebreite durchlaufende Gehäuseöffnung 20 aufweist, an die sich ein Hohlraum 21 anschließt, der wie die Gehäuseöffnung 20 quer über die gesamte Gehäusebreite verläuft. Der Hohlraum 21 ist groß genug, dass ein Benutzer seine Hände 3 durch die Gehäuseöffnung 20 vollständig in den Hohlraum 21 einschieben kann. Dies ist in der Seitenansicht des Handtrockners in Figur 3 angedeutet, wo der Teil eines Arms sowie eine Hand 3 des Benutzers teilweise in den Hohlraum 21 eingeschoben dargestellt sind.

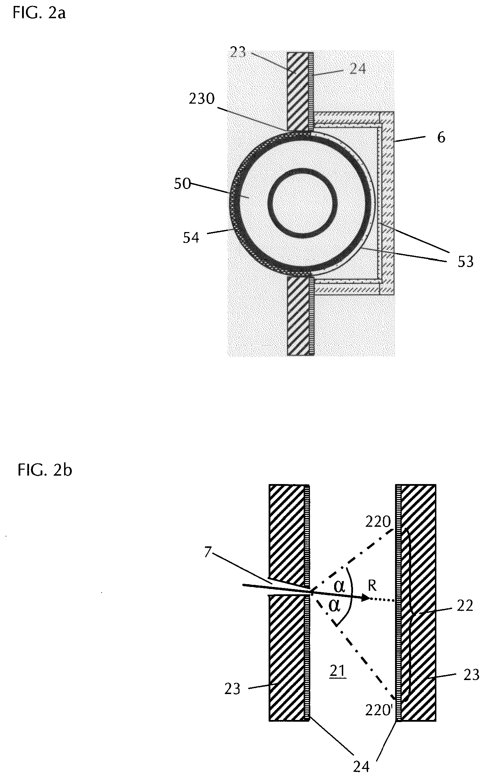

Wie in der Querschnittsdarstellung der Figur 2 erkennbar, ist im unteren Bereich des Gehäuses 2 ein Hohlraum vorhanden, in dem ein Lüfter 4 angeordnet ist, der Luft mit hoher Geschwindigkeit durch zwei Lüftungskanäle 70, 70' in Richtung auf 2 Flachdüsen 7, 7' fördert, die benachbart zur Gehäuseöffnung 20 auf gegenüberliegenden Seiten in den Hohlraum 21 einmünden. Aus den Flachdüsen 7, 7' wird Luft mit einer Geschwindigkeit von mindestens 15 m/s in Richtung auf die in den Hohlraum 21 eingeschobenen Hände 3 des Benutzers geblasen. Das Gebläse 4 tritt erst dann in Aktion, wenn die beiden Sensoren 8, 8' einen Gegenstand - üblicherweise die Hände eines Benutzers - im Spalt zwischen ihnen detektieren. Das Gebläse 4 wird dann für einen vorgegebenen Zeitraum in Betrieb gesetzt. Zudem werden auch die beiden Lampen 50, 50' angeschaltet, die Teil einer Vorrichtung 5 zur Erzeugung von UV-Strahlung sind. Die Vorrichtung 5 gibt UV-Strahlung mit einer Wellenlänge im Bereich von 200 bis 380 nm, von der maximal 20 % der Intensität in einem Wellenlängenbereich von 228 bis 380 nm liegt, in den Hohlraum ab.

Figur 2a zeigt den Teil Y der Vorrichtung 5 zur Erzeugung von UV-Strahlung in einer vergrößerten Darstellung. Die Lampenanordnung auf der gegenüberliegenden Seite des Hohlraums 21 ist entsprechend ausgebildet. Bei der Lampe 50 handelt es sich im gezeigten Beispiel um eine Krypton-Halogenid-Excimer-Barrierentladungslampe, konkret eine KrCl- oder KrBr-Excimer-Barrierentladungslampe, mit einem röhrenförmigen Lampenkolben. Dieser ist so angeordnet, dass er parallel zur Öffnungsfläche der Gehäuseöffnung 20 verläuft und gleichzeitig parallel zum Sensor 8 und zur Austrittsöffnung der Flachdüse 7, welche zwischen dem Lampenkolben und der Gehäuseöffnung 20 angeordnet sind. Die Flachdüse ist im konkreten Fall 25 cm lang. Der Lampenkolben 51 ist in einer ebenfalls 25 cm langen schlitzförmigen Öffnung 230 einer den Hohlraum 21 begrenzenden Seitenwand 23 angeordnet, und zwar derart, dass etwa ein Drittel des vom Lampenkolben eingenommenen Volumens innerhalb des Hohlraums 21 liegen, während die restlichen ungefähr 2 Drittel des Kolbenvolumens sich außerhalb des Hohlraums 21 befinden. Das teilweise Einschieben des Lampenkolbens 51 in den Hohlraum 21 sorgt dafür, dass die von der Lampe abgegebene Strahlung sämtliche Bereiche des Hohlraums 21 erreichen kann, ohne den für die Hände des Benutzers zur Verfügung stehenden Raum zu stark zu verkleinern oder die Lampe einem zu großen Beschädigungs- und Verschmutzungsrisiko auszusetzen. Für den Schutz der Lampe sorgt zusätzlich ein Fensterbauteils 6, dass den in den Hohlraum 21 hineinragenden Teil des Lampenkolbens 51 vollständig überdeckt und die schlitzförmige Öffnung 230 gegenüber dem Hohlraum 21 luft- und feuchtigkeitsdicht abschließt.

Der nicht in den Hohlraum 21 hineinragende Bereich des Lampenkolbens 51 wird von einem Reflektor 54 umgeben, dass hier die Form einer geschlitzten Metallhülse 540 aufweist, wobei die Schlitzöffnung 541 eine Strahlungsaustrittsöffnung zum Hohlraum 21 hin bildet (vergleiche auch Figuren 4a und 4b). Alternativ kann es sich bei dem Reflektor 54 auch um eine Metallbeschichtung handeln. Wird eine Metallhülse 540 verwendet, kann diese gleichzeitig auch als Außenelektrode der dielektrischen Barrierentladungslampe dienen. Im Beispiel der Figur 2 ist der in den Hohlraum 21 ragende Teil des Lampenkolbens 51 mit einem Filtermaterial 53 beschichtet, welches in der Lage ist, UV-Strahlung mit Wellenlängen im Bereich oberhalb von 228 nm aus der Strahlung herauszufiltern, welche in den Hohlraum emittiert wird. Alternativ oder zusätzlich kann Filtermaterial 53 auch auf dem Fensterbauteil 6 als Beschichtung aufgetragen sein, oder das Fensterbauteil 6 kann insgesamt aus einem Filtermaterial hergestellt werden.

Figur 2b verdeutlicht schematisch die Anordnung der Lampe 50' in Bezug auf die gegenüberliegende Flachdüse 7. Für die Lampe 50 und die gegenüberliegende Flachdüse 7' gilt das Entsprechende. Wie sich der Figur entnehmen lässt, ist die Flachdüse 7 leicht zum Inneren des Hohlraums 21 hin geneigt. Die Luft tritt mit einer Geschwindigkeit von mindestens 15 m/s als Luftvorhang entlang der mit dem Pfeil und der anschließenden gestrichelten Linie verdeutlichten Strömungsrichtung R in den Hohlraum 21 ein. Über die gesamte Breite der Flachdüse 7 gesehen, ergibt die im Querschnitt als Linie dargestellte Strömungsrichtung eine Ebene, ausgehend von der erfindungsgemäß ein Winkelbereich α definiert wird, der sich keilförmig in Richtung auf die gegenüberliegende Seitenwand 23' hin öffnet. Die Schnittfläche, die der keilförmige Bereich auf der Seitenwand 23' bildet, ergibt einen quer verlaufenden streifenförmigen Bereich 22 mit einem oberen Rand 220 und einem unteren Rand 220'. Innerhalb dieses Streifens wird erfindungsgemäß die Lampe 50' angeordnet, wobei der Winkel α 90° beträgt und sich gleichmäßig beidseitig der Strömungsrichtungsebene R aufteilt, also a/2 45° jeweils oberhalb und unterhalb der Ebene R liegen.

Wie sich Fig. 2, 2a und 2b weiter entnehmen lässt, sind die den Hohlraum begrenzenden Wände 23 und der Hohlraumboden mit einer Beschichtung 24 versehen. Diese enthält einen Fotokatalysator, konkret Titandioxid. Bei Bestrahlung mit UV-Strahlung bewirkt der Fotokatalysator die Bildung von Radikalen, beispielsweise OH-Radikalen aus Wasser, und Ozon, welche jeweils keimtötend wirken. Auf diese Weise wird die Ablagerung von Keimen an den Hohlraumwänden verhindert.

Figuren 4a und 4b zeigen 2 unterschiedliche Möglichkeiten der Anordnung des Lampenkolbens 51 in Bezug auf die Seitenwand 23 und den Hohlraum 21. Während in Figur 4a der Lampenkolben 51 nur sehr wenig in den Hohlraum 21 hineinragt, befinden sich in Figur 4b etwa 40 % des Volumens des Lampenkolbens 51 im Hohlraum 21. Je weiter der Lampenkolben in den Hohlraum 21 vorsteht, desto besser kann letzterer mit UV-Strahlung bestrahlt werden, wobei jedoch die Gefahr von Beschädigungen und Verunreinigungen steigt und mehr Platz im Hohlraum vom Lampenkolben und dem diesen abdeckenden Fensterbauteil 6 beansprucht wird. Figur 4a zeigt zudem ein anderes Beispiel einer Lampe 50, nämlich einer solchen, bei der die erzeugte Strahlung von einer Beschichtung aus Fluoreszenzmaterial 52 wie LaPO₄:Pr in die in den Hohlraum 21 abgegebene UV-Strahlung mit dem erhöhten Anteil an Strahlung mit Wellenlängen unter 228 nm umgewandelt wird. Beispielsweise kann es sich um eine Edelgas-Barrierentladungslampe handeln.

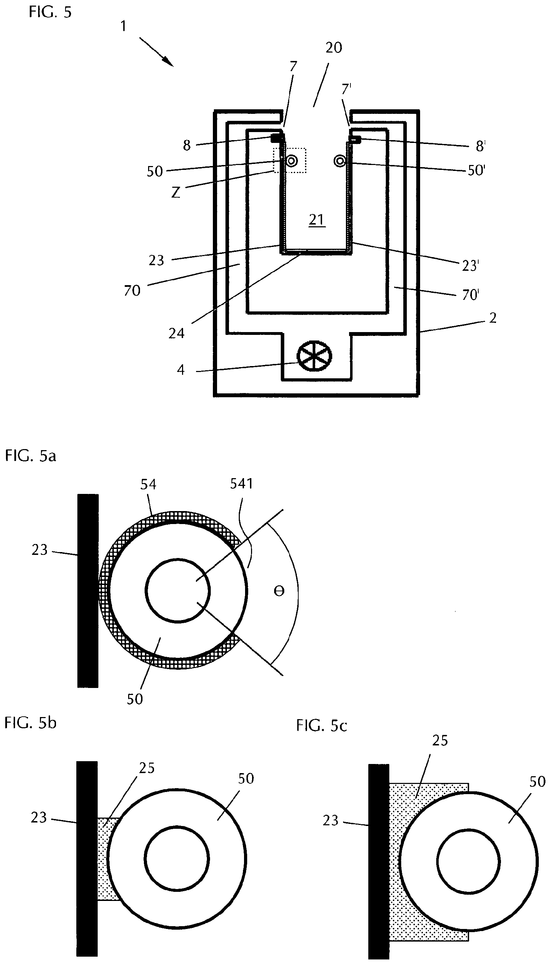

Figur 5 zeigt eine alternative Ausführungsform eines erfindungsgemäßen Handtrockners 1. Er unterscheidet sich von demjenigen der Figur 2 im Wesentlichen durch die Anordnung der Lampen 50, 50'. Diese befinden sich nun vollständig innerhalb des Hohlraums 21. Die röhrenförmigen Lampenkolben, die sich im Wesentlichen parallel zur Öffnung 20 erstrecken, haben zweckmäßig einen möglichst geringen Durchmesser, zum Beispiel 15 mm, um im Hohlraum 21 möglichst viel Platz für die Hände des Benutzers frei zu lassen.

Figuren 5a bis 5c zeigen unterschiedliche Möglichkeiten der Anordnung der Lampe 50 in einer Ausschnittvergrößerung des Bereichs Z der Figur 5. In Figur 5a ist der Lampenkolben der Lampe 50 von einem Reflektor 54 umgeben, der in Richtung zum Hohlraum 21 hin eine Strahlungsaustrittsöffnung 541 freilässt. Diese ermöglicht einen Austritt von UV-Strahlung in den Hohlraum 21 mit einem Öffnungswinkel Θ von mehr als 30°, hier konkret etwa 80°. Der Öffnungswinkel wird in Abhängigkeit von den Abmessungen des Handtrockners 1 so gewählt, dass eine ausreichende Bestrahlung des Hohlraums und eine möglichst vollständige Bestrahlung der Hände eines Benutzers erfolgen können.

Figuren 5b und 5c zeigen alternative Lampenanordnungen, bei denen die Lampe 50 jeweils mittels eines Befestigungsbauteils 25 an einer Seitenwand 23 des Hohlraums 21 befestigt ist. Über die Größe des Befestigungsbauteils 25 wird hier der Öffnungswinkel **Θ** vorgegeben. Im Fall der Figur 5b deckt das Befestigungsbauteil nur etwa 60° des Umfangs des Lampenkolbens ab, womit sich ein Öffnungswinkel von etwa 300° ergibt. Im Falle der Figur 5c bedeckt das Befestigungsbauteil etwa den halben Umfang des Lampenkolbens, woraus ein Öffnungswinkel von ca. 180° resultiert.

Die Wirksamkeit der Offenbarung wurde im Vergleich zu einem herkömmlichen Handtrocknungsverfahren untersucht. In dem Beispiel der Erfindung wurde ein Handtrockner mit dem in Figuren 1 und 2 beschriebenen Aufbau verwendet, wobei als Lampen 50, 50' jeweils eine KrCl-Excimer-Barrierentladungslampe mit einer Hauptwellenlänge von 222 nm eingesetzt wurde. Diese Lampen wurden jeweils mit etwa 20 W betrieben, wobei die Bestrahlungsintensität auf der Handoberfläche bei ungefähr 3 mW/cm² lag. Die Gesamtdosis der UV-Strahlung mit 222 nm während einer Trocknungszeit von etwa 5 Sekunden betrug im Durchschnitt 15 mJ/cm². Nach Ende der Trocknungszeit wurde von jeder Hand der Testperson mit käuflich erhältlichem Petrifilm der Firma 3M eine Probe abgezogen und entsprechend den Vorschriften der Firma inkubiert. Anschließend wurden die auf dem jeweiligen Petrifilm gefundenen Kolonien ausgezählt. Pro Testreihe wurden 2 Proben genommen. Aus dem Durchschnitt der Auszählung ergab sich das Testergebnis der Testreihe. Die Versuche umfassten folgende Testreihen:
1) Die Hände wurden mit Seife gründlich gewaschen und in dem Handtrockner ohne Einstrahlung von UV-Strahlung in den Hohlraum 21 getrocknet.
2) Die Hände wurden mit Seife gründlich gewaschen und in dem Handtrockner *mit* Einstrahlung von UV-Strahlung in den Hohlraum 21 getrocknet.
3) Die Hände wurden *nicht* gewaschen und in dem Handtrockner ohne Einstrahlung von UV-Strahlung in den Hohlraum 21 getrocknet.
4) Die Hände wurden *nicht* gewaschen und in dem Handtrockner *mit* Einstrahlung von UV-Strahlung in den Hohlraum 21 getrocknet.

Fotografien der beiden Proben, die pro Testreihe 1 bis 4 von den Handflächen genommen wurden, sind in Figuren 5a bis 5d gezeigt, wobei im linken Bereich der Abbildungen jeweils nur der halbe Petrifilm dargestellt ist. Figur 5a zeigt das Ergebnis der beiden Proben aus Testreihe 1, für die die Auszählung der Kolonien auf den Petrifilmen Ergebnisse von 24 und 13 Kolonien, im Durchschnitt 18,5 Kolonien, ergab. Figur 5b zeigt die Petrifilme aus Testreihe 2 mit zwei und einer Kolonie, im Durchschnitt also 1,5 Kolonien. Testreihe 3 ergab für die in Figur 5c gezeigten Petrifilme 480 und 812 Kolonien mit einem Durchschnitt von 646 Kolonien, Testreihe 4 schließlich für die in Figur 5d dargestellten Petrifilme 32 und 43 Kolonien, durchschnittlich also 37,5 Kolonien.

Die obigen Ergebnisse zeigen, dass grundsätzlich bereits mit gründlichem Händewaschen und -trocknen eine relativ gute Reinigung der Hände möglich ist, entsprechend einer Restkeimrate von etwa 3 %, verglichen mit Testreihe 3, bei der die Hände lediglich getrocknet wurden und keine Keimreduzierung stattfand. Testreihe 4 verdeutlicht, dass sich selbst bei ungewaschenen Händen allein aufgrund der Bestrahlung mit UV-Strahlung eine deutliche Reduktion der auf den Händen befindlichen Keime erreichen lässt, nämlich eine Keimrate von etwa 5 bis 8 % im Vergleich zur Testreihe 3. Die mit Abstand besten Ergebnisse, nämlich praktisch vollständig von Keimen befreite Hände mit einem Restkeimbestand von etwa 0,2 % im Vergleich zur Testreihe 3, wurden in Testreihe 2 erzielt, also durch gründliches Waschen der Hände und anschließendes Trocknen im erfindungsgemäßen Handtrockner mit Einstrahlung des erfindungsgemäß ausgewählten Wellenlängenbereichs der UV-Strahlung.

## Patentansprüche

1. Handtrockner (1) mit einem Gehäuse (2), in dem ein durch eine Gehäuseöffnung (20) von außen zugänglicher Hohlraum (21) zur Aufnahme mittels einer Luftströmung im Hohlraum (21) zu trocknender Hände (3) ausgebildet ist, und mit einer Vorrichtung (4) zur Erzeugung der Luftströmung sowie einer Vorrichtung (5) zur Erzeugung von UV-Strahlung, welche wenigstens eine im ultravioletten Wellenlängenbereich emittierende Lampe (50) umfasst, die derart im Gehäuse (2) angeordnet ist, dass sie UV-Strahlung in den Hohlraum (21) abgibt, dadurch ge ke n nzeich net,
dass die Vorrichtung (5) zur Erzeugung von UV-Strahlung derart ausgebildet ist, dass die in den Hohlraum (21) abgegebene UV-Strahlung mit einer Wellenlänge im Bereich von 228 bis 380 nm eine Intensität von maximal 20 % der Intensität der in den Hohlraum (21) abgegebenen UV-Strahlung mit einer Wellenlänge im Bereich von 200 bis 380 nm besitzt.

2. Handtrockner nach Anspruch 1,
**dadurch gekennzeichnet,**
**dass** die in den Hohlraum (21) abgegebene UV-Strahlung mit einer Wellenlänge im Bereich von 228 bis 380 nm eine Intensität von maximal 15 %, bevorzugt maximal 10 % und insbesondere maximal 7 %, der Intensität der in den Hohlraum (21) abgegebene UV-Strahlung mit einer Wellenlänge im Bereich von 200 bis 380 nm besitzt.

3. Handtrockner nach Anspruch 1 oder 2,
**dadurch gekennzeichnet,**
**dass** die wenigstens eine Lampe (50) ausgewählt ist aus
- einer Lampe (50) mit einer Haupt-Emissionswellenlänge von unter 228 nm, bevorzugt einer dielektrischen Barrierentladungslampe und insbesondere einer KrCl- oder KrBr-Excimer-Barrierentladungslampe,
- einer Lampe, welche ein Fluoreszenzmaterial (52) anregende Strahlung erzeugt, wobei die durch Anregung des Fluoreszenzmaterials emittierte Strahlung eine Hauptwellenlänge unter 228 nm aufweist, wobei die Lampe bevorzugt eine Edelgas-Barrierentladungslampe ist,
- einer Lampe (50) in Kombination mit einem Filtermaterial (53), welches UV-Strahlung mit einer Wellenlänge im Bereich von 228 nm bis 300 nm und bevorzugt 230 bis 260 nm herausfiltert.

4. Handtrockner nach einem der Ansprüche 1 bis 3,
**dadurch gekennzeichnet,**
**dass** ein Lampenkolben (51) der Lampe (50) so im Gehäuse (2) angeordnet ist, dass ein Teil desselben in den Hohlraum (21) hineinragt, wobei ein überwiegender Teil des Lampenkolbens (51) sich außerhalb des Hohlraums (21) befindet.

5. Handtrockner nach einem der Ansprüche 1 bis 3,
**dadurch gekennzeichnet,**
**dass** ein Lampenkolben (51) der Lampe (50) vollständig im Hohlraum (21) angeordnet ist.

6. Handtrockner nach Anspruch 4 oder 5,
**dadurch gekennzeichnet,**
**dass** der im Hohlraum (21) befindliche Teil des Lampenkolbens (51) von einem Fensterbauteil (6) gegen den Hohlraum (21) abgegrenzt ist.

7. Handtrockner nach einem der Ansprüche 4 bis 6,
**dadurch gekennzeichnet,**
**dass** das Filtermaterial (53) auf dem Lampenkolben (51) und/oder dem Fensterbauteil (6) angebracht ist oder das Fensterbauteil (6) aus dem Filtermaterial (53) besteht.

8. Handtrockner nach einem der Ansprüche 4 bis 7,
**dadurch gekennzeichnet,**
**dass** der Lampenkolben (51) unter Freilassung einer Strahlungsaustrittsöffnung (541) von einem Reflektor (54) umgeben ist, welcher die UV-Strahlung mit einer Wellenlänge von unter 228 nm in Richtung auf den Hohlraum (21) reflektiert.

9. Handtrockner nach Anspruch 8,
**dadurch gekennzeichnet,**
**dass** der Reflektor (54) eine Metallbeschichtung des Lampenkolbens (51) oder eine Metallhülse (540) ist, die unter Freilassung der Strahlungsaustrittsöffnung (541) auf der Außenseite des Lampenkolbens (51) angeordnet ist und bevorzugt gleichzeitig eine Außenelektrode der Lampe (50) darstellt.

10. Handtrockner nach Anspruch 8 oder 9,
**dadurch gekennzeichnet,**
**dass** die Strahlungsaustrittsöffnung (541) ausgebildet ist, Strahlung mit einem Öffnungswinkel (Θ) von mindestens 30° in Richtung des Hohlraums (21) durchzulassen.

11. Handtrockner nach einem der Ansprüche 1 bis 10,
**dadurch gekennzeichnet,**
**dass** zwei, insbesondere röhrenförmige, Lampen (50, 50') auf gegenüberliegenden Seiten des Hohlraums (21) angeordnet sind.

12. Handtrockner nach einem der Ansprüche 1 bis 11,
**dadurch gekennzeichnet,**
**dass** wenigstens eine Flachdüse (7), insbesondere je eine Flachdüse (7, 7') auf gegenüberliegenden Seiten des Hohlraums (21), in einem der Gehäuseöffnung (20) benachbarten Bereich vorhanden und ausgebildet ist, eine Luftströmung mit einer Geschwindigkeit von mindestens 15 m/s in den Hohlraum (21) abzugeben.

13. Handtrockner nach Anspruch 12,
dadurch gekennze ich net,
dass die wenigstens eine Lampe (50, 50') innerhalb eines streifenförmigen Bereichs (22) angeordnet ist, der sich auf einer den Hohlraum (21) begrenzenden Seitenwand (23) gegenüber einer Flachdüse (7, 7') befindet und dessen Ränder (220, 220') den Schnittlinien entsprechend, mit denen die Grenzlinien eines Winkelbereichs (α) von ± 45°, ausgehend von der Luftaustrittsrichtung (R) aus der Flachdüse (7, 7'), die Seitenwand (23) schneiden.

14. Handtrockner nach einem der Ansprüche 1 bis 13,
**dadurch gekennzeichnet,**
**dass** zumindest ein Teil der den Hohlraum (21) begrenzenden Wände wenigstens bereichsweise mit einem Fotokatalysator versehen ist.

15. Handtrockner nach Anspruch 14,
**dadurch gekennzeichnet,**
**dass** der Fotokatalysator Titandioxid umfasst, das in einer auf die Oberfläche der Wände aufgetragenen Beschichtung (24) enthalten oder in das Material der Wände eingearbeitet ist.

## Claims

1. A hand dryer (1) with a housing (2) in which a cavity (21) accessible from outside through a housing opening (20) is formed for accommodating hands (3) to be dried by means of an airflow, and with a device (4) for generating the airflow, as well as a device (5) for generating UV radiation comprising at least one lamp (50) that emits light in the ultraviolet wavelength range, which is arranged in the housing (2) in such a manner that it emits UV radiation into the cavity (21),
**characterized in that**
the device (5) for generating UV radiation is configured in such a manner that the UV radiation emitted into the cavity (21) with a wavelength in the range from 228 to 380 nm has a maximum intensity of 20 % of the intensity of the UV radiation emitted into the cavity (21) with a wavelength in the range from 200 to 380 nm.

2. The hand dryer according to claim 1,
**characterized in that**
the UV radiation emitted into the cavity (21) with a wavelength in the range from 228 to 380 nm has a maximum intensity of 15 %, preferably a maximum of 10 %, and especially a maximum of 7 %, of the intensity of the UV radiation emitted into the cavity (21) with a wavelength in the range from 200 to 380 nm.

3. The hand dryer according to claim 1 or 2,
**characterized in that**
the at least one lamp (50) is selected from
- a lamp (50) with a main emission wavelength below 228 nm, preferably a dielectric barrier discharge lamp and in particular a KrCl or KrBr excimer barrier discharge lamp;
- a lamp that generates radiation which excites a fluorescent material (52), the radiation emitted by excitation of the fluorescent material having a main wavelength below 228 nm, the lamp preferably being a noble gas barrier discharge lamp;
- a lamp (50) in combination with a filter material (53) that filters out UV radiation with a wavelength in the range from 228 nm to 300 nm and preferably 230 to 260 nm.

4. The hand dryer according to one of claims 1 to 3,
**characterized in that**
a lamp bulb (51) of the lamp (50) is arranged in the housing (2) in such a manner that a part of the same projects into the cavity (21) while a predominant part of the lamp bulb (51) is located outside the cavity (21).

5. The hand dryer according to one of claims 1 to 3,
**characterized in that**
a lamp bulb (51) of the lamp (50) is arranged completely inside the cavity (21).

6. The hand dryer according to claim 4 or 5,
**characterized in that**
the part of the lamp bulb (51) located inside the cavity (21) is separated from the cavity (21) by a window component (6).

7. The hand dryer according to one of claims 4 to 6,
**characterized in that**
the filter material (53) is attached to the lamp bulb (51) and/or the window component (6), or the window component (6) consists of the filter material (53).

8. The hand dryer according to one of claims 4 to 7,
**characterized in that**
the lamp bulb (51) is surrounded by a reflector (54) which leaves open a radiation emission opening (541), said reflector reflecting the UV radiation with a wavelength below 228 nm towards the cavity (21).

9. The hand dryer according to claim 8,
**characterized in that**
the reflector (54) is a metal coating of the lamp bulb (51) or a metal sleeve (540) which is arranged on the exterior of the lamp bulb (51) and which leaves free the radiation emission opening (541), and preferably simultaneously constitutes an outer electrode of the lamp (50).

10. The hand dryer according to claim 8 or 9,
**characterized in that**
the radiation emission opening (541) is designed to allow radiation to pass towards the cavity (21) at an opening angle (Θ) of at least 30°.

11. The hand dryer according to one of claims 1 to 10,
**characterized in that**
two lamps (50, 50'), in particular with a tubular shape, are arranged on opposite sides of the cavity (21).

12. The hand dryer according to one of claims 1 to 11,
**characterized in that**
at least one flat nozzle (7), in particular one flat nozzle (7, 7') on each of the opposite sides of the cavity (21), is provided in an area adjacent to the housing opening (20) and is designed to discharge an airflow into the cavity (21) at a velocity of at least 15 m/s.

13. The hand dryer according to claim 12,
**characterized in that**
the at least one lamp (50, 50') is arranged within a strip-shaped area (22) which is located opposite a flat nozzle (7, 7') on a sidewall (23) delimiting the cavity (21) and which has edges (220, 220') that correspond to the section lines with which the boundaries of an angular range (α) of ± 45°, based on the direction (R) of the air exiting the flat nozzle (7, 7'), intersect the sidewall (23).

14. The hand dryer according to one of claims 1 to 13,
**characterized in that**
at least areas of at least a part of the walls delimiting the cavity (21) are provided with a photocatalyst.

15. The hand dryer according to claim 14,
**characterized in that**
the photocatalyst comprises titanium dioxide, which is contained in a coating (24) applied to the surface of the walls or incorporated into the wall material.

## Revendications

1. Sèche-mains (1) avec un boîtier (2) dans'lequel est formée une cavité (21) accessible de l'extérieur par une ouverture (20) du boîtier afin d'accueillir les mains (3) à sécher au moyen d'un flux d'air et avec un dispositif (4) destiné à générer le flux d'air, ainsi qu'un dispositif (5) destiné à générer un rayonnement UV comprenant au moins une lampe (50) qui émet de la lumière dans la gamme de longueurs d'onde UV, qui est installé dans le boîtier (2) de telle façon qu'il émette un rayonnement UV dans la cavité (21),
**caractérisé en ce que**
le dispositif (5) destiné à générer le rayonnement UV est configuré de telle façon que le rayonnement UV émis dans la cavité (21) avec une longueur d'onde dans la gamme comprise entre 228 et 380 nm possède une intensité maximale égale à 20 % de l'intensité du rayonnement UV émis dans la cavité (21) avec une longueur d'onde dans la gamme de 200 à 380 nm.

2. Sèche-mains selon la revendication 1,
**caractérisé en ce que**
le rayonnement UV émis dans la cavité (21) avec une longueur d'onde dans la gamme comprise entre 228 et 380 nm possède une intensité maximale égale à 15 %, de manière préférée une intensité maximale égale à 10 %, et tout spécialement une intensité maximale égale à 7 % de l'intensité du rayonnement UV émis dans la cavité (21) avec une longueur d'onde dans la gamme de 200 à 380 nm.

3. Sèche-mains selon la revendication 1 ou 2,
**caractérisé en ce que**
ladite au moins une lampe (50) est choisie parmi :
- une lampe (50) avec une longueur d'onde d'émission principale inférieure à 228 nm, de manière préférée une lampe à décharge à barrière diélectrique, et en particulier une lampe à décharge à barrière à excimère KrCl ou KrBr;
- une lampe qui génère un rayonnement qui excite un matériau fluorescent (52), le rayonnement émis par l'excitation du matériau fluorescent ayant une longueur d'onde principale inférieure à 228 nm, la lampe étant de manière préférée une lampe à décharge à barrière à gaz noble;
- une lampe (50) combinée à une matière filtrante (53) qui filtre le rayonnement UV avec une longueur d'onde dans une gamme comprise entre 228 nm et 300 nm, et de manière préférée entre 230 et 260 nm.

4. Sèche-mains selon l'une quelconque des revendications 1 à 3,
**caractérisé en ce que**
une ampoule (51) de la lampe (50) est disposée dans le boîtier (2) d'une façon telle qu'une partie de celle-ci dépasse dans la cavité (21), une majeure partie de l'ampoule (51) de la lampe étant située à l'extérieur de la cavité (21).

5. Sèche-mains selon l'une quelconque des revendications 1 à 3,
**caractérisé en ce que**
une ampoule (51) de la lampe (50) est disposée en totalité à l'intérieur de la cavité (21).

6. Sèche-mains selon la revendication 4 ou 5,
**caractérisé en ce que**
la partie de l'ampoule (51) située à l'intérieur de la cavité (21) est séparée de la cavité (21) par un élément de vitrage (6).

7. Sèche-mains selon l'une quelconque des revendications 4 à 6,
**caractérisé en ce que**
la matière filtrante (53) est apposée sur l'ampoule (51) de la lampe et/ou sur l'élément de vitrage (6), ou bien l'élément de vitrage (6) est constitué par la matière filtrante (53).

8. Sèche-mains selon l'une quelconque des revendications 4 à 7,
**caractérisé en ce que**
l'ampoule (51) de la lampe est entourée d'un réflecteur (54) qui laisse ouverte une ouverture (541) pour l'émission du rayonnement, ledit réflecteur réfléchissant le rayonnement UV vers la cavité (21) avec une longueur d'onde inférieure à 228 nm.

9. Sèche-mains selon la revendication 8,
**caractérisé en ce que**
le réflecteur (54) est un revêtement métallique de l'ampoule (51) de la lampe ou un manchon métallique (540) qui est disposé sur l'extérieur de l'ampoule (51) de la lampe et qui laisse libre l'ouverture (541) pour l'émission du rayonnement, et de manière préférée constitue simultanément une électrode extérieure de la lampe (50).

10. Sèche-mains selon la revendication 8 ou 9,
**caractérisé en ce que**
l'ouverture (541) pour l'émission du rayonnement est prévue pour permettre au rayonnement de traverser la cavité (21) selon un angle d'ouverture (**θ**) d'au moins 30°.

11. Sèche-mains selon l'une quelconque des revendications 1 à 10,
**caractérisé en ce que**
deux lampes (50, 50'), en particulier présentant une forme tubulaire, sont disposées sur des côtés opposés de la cavité (21).

12. Sèche-mains selon l'une quelconque des revendications 1 à 11,
**caractérisé en ce que**
au moins une buse plate (7), en particulier une buse plate (7, 7') sur chacun des côtés opposés de la cavité (21), est prévue dans une zone adjacente à l'ouverture (20) du boîtier et est conçue pour décharger un flux d'air dans la cavité (21) à une vitesse d'au moins 15 m/s.

13. Sèche-mains selon la revendication 12,
**caractérisé en ce que**
ladite au moins une lampe (50, 50') est placée dans une zone (22) en forme de bande qui est située à l'opposé d'une buse plate (7, 7') sur une paroi latérale (23) délimitant la cavité (21) et qui présente des bords (220, 220') qui correspondent aux lignes de coupe avec lesquelles les lignes de délimitation d'une plage angulaire (α) de ± 45°, sur la base de la direction (R) de l'air sortant de la buse plate (7, 7'), intersectent la paroi latérale (23).

14. Sèche-mains selon l'une quelconque des revendications 1 à 13,
**caractérisé en ce que**
au moins des zones d'au moins une partie des parois délimitant la cavité (21) sont pourvues d'un photocatalyseur.

15. Sèche-mains selon la revendication 14,
**caractérisé en ce que**
le photocatalyseur comprend du dioxyde de titane qui est contenu dans un revêtement (24) appliqué à la surface des parois ou incorporé dans le matériau des parois.
